# EUROPÄISCHE PATENTSCHRIFT

(11) **EP 1 574 178 B1**
(45) Veröffentlichungstag und Bekanntmachung des Hinweises auf die Patenterteilung: **26.09.2018**
(21) Anmeldenummer: 05101158.3
(22) Anmeldetag: 16.02.2005
(51) Int. Cl.: H04N 7/15, G16H 80/00

(54) **Medizinisches Behandlungssystem**
Medical treatment system
Système de traitement médical

(30) Priorität: 13.03.2004 DE 102004012447
(43) Veröffentlichungstag der Anmeldung: 14.09.2005
(73) Patentinhaber: B. Braun Avitum AG, 34212 Melsungen (DE)
(72) Erfinder: Moll, Stefan, 34212 Melsungen (DE); Bock, Gerhard, 36289 Friedewald (DE); Möller, Dirk, 34326 Altmorschen (DE)
(74) Vertreter: Winter, Brandl, Fürniss, Hübner, Röss, Kaiser, Polte - Partnerschaft mbB

(56) Entgegenhaltungen:
- WO-A-01/72027
- WO-A-01/86575
- DE-A1- 10 163 095
- FR-A1- 2 806 561
- US-A- 5 544 649
- US-A- 5 801 755

## Beschreibung

Die Erfindung betrifft ein medizinisches Behandlungssystem mit mindestens einem Behandlungsplatz für die Hämodialyse, der eine Dialysemaschine als Behandlungsgerät, eine Videokamera, einen Bildschirm und eine Gegensprechvorrichtung aufweist, und mindestens einem Arztplatz, der einen Computer, einen Bildschirm und eine Gegensprechvorrichtung aufweist, wobei der Computer des Arztplatzes mit dem Behandlungsgerät über eine Datenübertragungsverbindung zur Übertragung von Behandlungsdaten kommuniziert.

Es gibt medizinische Behandlungen, die an einem Patienten durchgeführt werden, ohne dass die ständige Anwesenheit eines Arztes am Behandlungsplatz erforderlich ist. Hierzu gehören extrakorporale Blutbehandlungen, beispielsweise die Blutdialyse. Bei der Blutdialyse ist die ständige Anwesenheit des Arztes beim Patienten nicht erforderlich, jedoch sollte der Arzt, wenn kritische Situationen auftreten, unverzüglich eingreifen können.

Eine wesentliche Leistung des Arztes ist die Erfassung des Patientenzustandes durch die Arztvisite, um daraus die Therapie abzuleiten. Für die Arztvisite sind der Blickkontakt und das Gespräch mit dem Patienten sowie Informationen über die Behandlungsparameter und Laborwerte wichtig. Technisch erfüllt werden können diese Bedürfnisse durch Videokonferenz, elektronische Patientenakte, Vernetzung der Behandlungsgeräte und einen interaktiven Bildschirm am Behandlungsgerät.

Bei einer Fernvisite mit Kommunikation über Videobilder und Gegensprechgeräte besteht die Möglichkeit der Verwechslung des Patienten. Der Arzt, der sich an einem zentralen Arztplatz befindet, kann zwar die Behandlungsdaten aus dem Computer eines entfernt angeordneten Behandlungsplatzes abrufen, jedoch besteht die Gefahr, dass die abgerufenen Daten einem falschen Patienten zugeordnet werden. Selbst im Falle einer Videokonferenz, bei der der Arzt den Patienten auf einem Bildschirm sieht, besteht die Gefahr von Verwechslungen, wenn der Arzt den Patienten nicht persönlich kennt.

Verwandter Stand der Technik ist aus den US-Patenten US 5,544,649 und US 5,801,755 sowie FR 2 806 561 A1 bekannt. Hierin sind medizinische Anwendungsfälle für eine interaktive Kommunikation offenbart, bei der ein Arzt mit einem Patienten, der sich etwa zu Hause befindet, über eine Videokonferenz kommunizieren kann.

Der Erfindung liegt die Aufgabe zugrunde, ein medizinisches Behandlungssystem anzugeben, das eine Fernvisite durch den Arzt über Telekommunikation ermöglicht und eine hohe Sicherheit der korrekten Zuordnung von Behandlungsparametern, Videobild und Sprache zu dem richtigen Patienten bietet.

Das medizinische Behandlungssystem nach der vorliegenden Erfindung ist durch den Patentanspruch 1 definiert.

Dadurch, dass Video- und Audiosignale über die Datenübertragungsverbindung übertragen werden, die auch die Behandlungsdaten überträgt, ist sichergestellt, dass der Arzt an seinem Bildschirm stets den richtigen Patienten sieht, dessen Behandlungsdaten er abrufen und ggf. verändern kann. Somit ist eine eindeutige und feste Zuordnung zwischen der Person des Patienten, den Behandlungsdaten, den Videobildern und der Sprache sichergestellt. Die Videokamera des Behandlungsplatzes ist an dem betreffenden Behandlungsplatz bzw. dem Behandlungsgerät fest installiert, ebenso wie der Anschluss für die Gegensprechvorrichtung.

Der Bildschirm des Behandlungsplatzes kann zugleich als Monitor des Behandlungsgerätes dienen, um die Behandlungsdaten anzuzeigen, bzw. zu verändern. In diesem Fall ist nur ein einziger Bildschirm am Behandlungsplatz erforderlich.

Das erfindungsgemäße medizinische Behandlungssystem eignet sich für Heimpatienten oder kleinere Satellitenzentren, die von einem Hauptzentrum aus betreut und überwacht werden. Durch Etablierung patientennaher Satellitenzentren können Patientenfahrten eingespart werden. Die Behandlungsparameter, die vom Arzt bestimmt werden, können dem Pflegepersonal über das Datennetz mitgeteilt werden, indem sie auf dem Bildschirm des Behandlungsplatzes angezeigt werden.

An dem Arztplatz kann eine zweite Videokamera angeordnet sein, die mit dem Bildschirm des Behandlungsplatzes über die Datenübertragungsverbindung oder einem diesem fest zugeordneten Kanal kommuniziert. Auf diese Weise kann dem Patienten bzw. der Schwester am Behandlungsplatz das Bild des Arztes sichtbar gemacht werden, so dass eine echte Videokonferenz zwischen ihnen durchgeführt werden kann.

Die Übertragung von Daten, Bildern und Sprache erfolgt über die üblichen Datennetze, zu denen sowohl lokale als auch öffentliche Datennetze gehören. Vorzugsweise werden Videosignale und Audiosignale in digitalisierter Form über das Datennetz übertragen. Die Übertragung kann sowohl drahtgebunden als auch drahtlos erfolgen.

Die einfachste und sicherste Zuordnung der Videokamera und der Gegensprechvorrichtung zu dem Behandlungsgerät erfolgt dadurch, dass die Videokamera und der Anschluss für die Gegensprechvorrichtung in das Behandlungsgerät integriert sind. In diesem Fall ist eine Verwechslung völlig unmöglich. Unter einer Gegensprechvorrichtung ist eine Sprech- und Hörvorrichtung zu verstehen wie sie beispielsweise als Headset bekannt ist. Eine solche Gegensprechvorrichtung hat einen Ohrhörer und ein Mikrofon. Sie bildet eine bidirektionale Schallwandvorrichtung.

Vorzugsweise ist die Videokamera des Behandlungsplatzes von dem Arztplatz aus fernsteuerbar, um auf verschiedene Ziele gerichtet werden zu können. Am Arztplatz befindet sich eine Positionssteuereingabe für die Behandlungsplatzkamera. Auf diese Weise kann der Arzt die Videokamera derart steuern, dass diese beispielsweise auf das Gesicht des Patienten oder auf den Shunt, d.h. die Austrittsstelle der Blutleitung aus dem Patientenkörper, gerichtet wird. Der Arzt kann auch die Umgebung des Patienten bzw. die Schwester beobachten.

Es kann vorgesehen sein, dass die Videokamera des Behandlungsplatzes eine Vorzugsrichtung aufweist, die sie einnimmt, wenn keine manuelle Steuerung der Richtung der Videokamera vom Arztplatz aus erfolgt. Diese Vorzugsrichtung kann beispielsweise das Gesicht des Patienten sein oder der Shunt.

Gemäß einer bevorzugten Ausgestaltung der Erfindung ist am Patientenplatz eine Vorrichtung zur Personenidentifikation fest installiert, die eine den Patienten kennzeichnende Identifizierung an den Computer des Behandlungsgerätes liefert. Diese Vorrichtung zur Personenidentifikation kann beispielsweise ein Magnetstreifenleser oder ein Chip-Lesegerät enthalten, das von einem Datenträger die Patientendaten liest und sie dem betreffenden Behandlungsplatz für die Dauer der Behandlung zuordnet. Es kann sich auch um eine Vorrichtung handeln, die biometrische Daten des Patienten ermittelt und auf diese Weise die Personenidentifikation vornimmt.

Wenn am Patientenplatz mehrere Geräte vorhanden sind, die unabhängig voneinander mobil sind und fortbewegt werden können, sollte jedes dieser Geräte eine Vorrichtung zur Personenidentifikation enthalten. Vorzugsweise sind alle Geräte eines Patientenplatzes fest miteinander verbunden oder einander fest zugeordnet, so dass sie von dem Pflegepersonal nicht ausgetauscht werden können. In diesem Fall ist nur eine Vorrichtung zur Personenidentifikation erforderlich.

Im Folgenden werden unter Bezugnahme auf die Zeichnungen Ausführungsbeispiele der Erfindung näher erläutert. Die Einzelheiten dieser Ausführungsbeispiele beschränken nicht den Schutzbereich der Erfindung. Dieser wird vielmehr durch die Patentansprüche bestimmt.

Es zeigen:
Fig. 1: Eine erste Ausführungsform des Behandlungssystems mit Behandlungsplatz und Arztplatz und
Fig. 2: eine modifizierte Ausführungsform des Behandlungsplatzes.

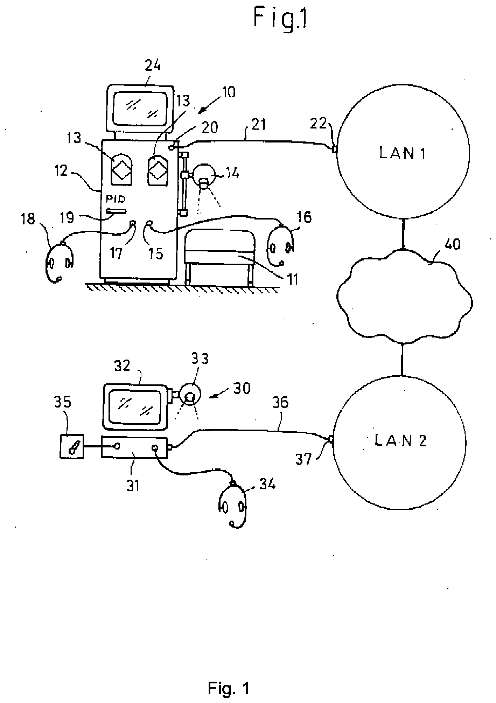

Das medizinische Behandlungssystem weist zahlreiche Behandlungsplätze auf, von denen ein Behandlungsplatz 10 exemplarisch in Figur 1 dargestellt ist. Bei dem hier beschriebenen Behandlungsplatz handelt es sich um einen Platz für die Hämodialysebehandlung, die die am meisten verbreitete Nierenersatztherapie bildet. Es gibt Patienten, die einer pflegerischen und ärztlichen Bereitschaft bedürfen, und solche, die die Behandlung mit pflegerischem Personal ohne die Anwesenheit eines Arztes durchführen, sowie Patienten, die die Behandlung alleine durchführen.

Der Behandlungsplatz 10 enthält eine Patientenliege 11, die neben einem Behandlungsgerät 12 aufgestellt ist. Das Behandlungsgerät 12 ist eine Dialysemaschine mit Blutpumpen 13 und einem (nicht dargestellten) Schlauchsystem für den extrakorporalen Anschluss des Blutkreislaufs eines Patienten. Zu dem Behandlungsgerät 12 gehört ein Bildschirm 24. Dieser ist hier als Touch-Screen ausgeführt, so dass keine zusätzliche Eingabevorrichtung in Form einer Tastatur erforderlich ist.

Das Behandlungsgerät 12 weist ferner eine Videokamera 14 auf, die fest mit dem Gehäuse des Behandlungsgerätes verbunden ist und hier seitlich von dem Gehäuse absteht. Die Videokamera 14 ist mit einem (nicht dargestellten) mehrachsigen Antrieb versehen, der ferngesteuert betätigt werden kann, um die Ausrichtung der Videokamera 14 zu verändern. So kann die Videokamera 14 beispielsweise auf das Gesicht des Patienten gerichtet werden oder auf den Shunt am Arm des Patienten. Auch der Zoom der Videokamera ist ferngesteuert.

An dem Gehäuse des Behandlungsgerätes 12 befindet sich eine Anschlussbuchse 15 für den Anschluss einer Gegensprechvorrichtung 16 und einer Anschlussbuchse 17 für den Anschluss einer zweiten Gegensprechvorrichtung 18. Die Gegensprechvorrichtungen 16 und 18 sind Headsets mit einem den Kopf übergreifenden Bügel, der zwei Kopfhörermuscheln trägt, und einem vor dem Mund des Benutzers angeordneten, am Bügel befestigten Mikrofon.

Schließlich ist an dem Behandlungsgerät 12 eine Vorrichtung 19 zur Personenidentifikation PID vorgesehen. Diese besteht hier aus einem Schlitz eines Kartenlesegerätes. Der Patient erhält eine Identifikationskarte im Scheckkartenformat, deren Daten maschinenlesbar sind und in der Vorrichtung 19 gelesen werden. Zu diesen Daten gehören u.a. der Name des Patienten und ggf. auch die an dem Behandlungsgerät 12 einzustellenden Behandlungsparameter.

Die Vorrichtung 19 zur Personenidentifikation kann auch zur Identifizierung und Speicherung der Bedienungsperson (Krankenschwester) dienen. Hierzu führt die Bedienungsperson eine maschinenlesbare Karte in den Schlitz der Vorrichtung ein, woraufhin in dem Computer des Behandlungsgerätes 12 die betreffende Person gespeichert wird. Diese Speicherung dient zur Dokumentation von Eingabe- und Bedienungsvorgängen, um nachweisen zu können, wer diese Vorgänge ausgeführt hat. Die Vorrichtung 19 gibt anschließend die Identifizierungskarte an die betreffende Person wieder zurück.

Das Behandlungsgerät 12 enthält einen (nicht separat dargestellten) Computer, der einerseits das Behandlungsgerät steuert und andererseits mit dem Bildschirm 24 vernetzt ist. Der Bildschirm als Touch-Screen bildet sowohl die Eingabevorrichtung als auch die Displayvorrichtung des Computers.

Der Computer weist einen Kommunikationsanschluss 20 auf, der über eine Datenleitung 21 mit einem Netzwerkanschluss 22 eines lokalen Netzwerks LAN1 verbunden ist. Das lokale Netzwerk ist beispielsweise ein internes Datennetz eines Dialyse-Subzentrums. An dieses lokale Datennetz sind mehrere Behandlungsgeräte angeschlossen, die jeweils einem Behandlungsplatz zugeordnet sind.

An einem von dem Behandlungsplatz 10 entfernten Ort, z.B. in einem Hauptzentrum, befindet sich der Arztplatz 30. Dieser weist einen Computer 31 mit Bildschirm 32 auf. Ferner ist eine Videokamera 33 vorgesehen, die hier am Bildschirm oder am Computer befestigt ist und auf das Gesicht des am Arztplatz sitzenden Arztes gerichtet ist. Der Computer 31 ist mit einer Gegensprechvorrichtung 34 verbunden, die am Kopf des Arztes befestigt wird. Außerdem ist der Computer 31 mit einer Steuervorrichtung 35, die einen Positionssteuerhebel aufweist, verbunden. Die Steuervorrichtung 35 dient zur Bewegungssteuerung der Videokamera 14 des Patientenplatzes.

Der Computer 31 des Arztplatzes ist über eine Datenleitung 36 mit einem Netzwerkanschluss 37 eines lokalen Netzwerks LAN2 verbunden. Die lokalen Netzwerke LAN1 und LAN2 sind mit einem allgemeinen Datennetz 40 verbunden, beispielsweise dem Telefonnetz oder dem Internet.

Die für die Datenverbindung erforderlichen Server, Router und ähnliche Einrichtungen sind aus Gründen der Übersichtlichkeit nicht dargestellt.

Der Patient gibt zu Beginn der Behandlung seine Identifizierung in den Computer des Behandlungsgerätes 12 ein, beispielsweise durch Einstecken einer entsprechenden Karte in die Vorrichtung 19. Der Arzt kann vom Arztplatz 30 aus die Verbindung zu einzelnen Behandlungsplätzen 10 bzw. Patienten aufnehmen. Er kann über die Gegensprechvorrichtungen sowohl mit dem Patienten sprechen als auch mit der zuständigen Pflegeperson. Dabei kann er die Videokamera 14 mit Hilfe der Steuervorrichtung 35 in die gewünschte Position bringen, um sich vom Zustand des Patienten und auch von dem ordnungsgemäßen Anschluss der Schläuche zu überzeugen. Auf dem Bildschirm 24 kann er darüber hinaus die Behandlungsdaten sowie die an dem Behandlungsgerät 12 eingestellten Werte aufrufen.

Das medizinische Behandlungssystem ist geeignet
- die Visualisierung der elektronischen Patientenakte am Arztplatz vorzunehmen,
- eine Behandlungsverschreibung eines Patienten elektronisch zuzuordnen,
- eine Videokonferenz mit einem Patienten am vom Arztplatz entfernten Ort durchzuführen,
- eine Überwachung des Patientenzustandes vom entfernten Ort vorzunehmen.

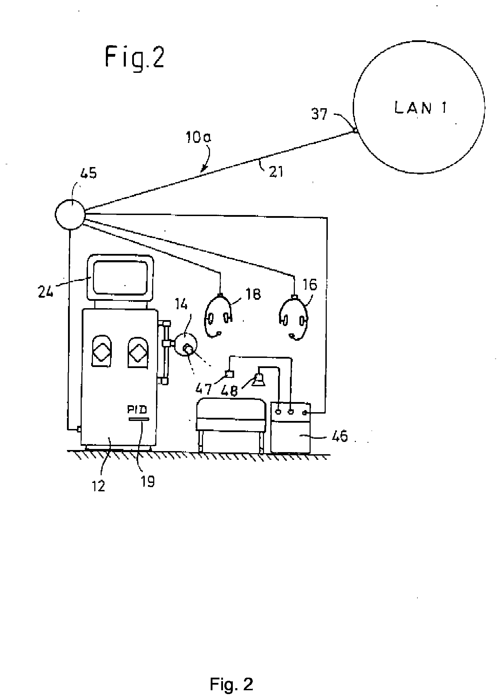

Das Ausführungsbeispiel von Fig. 2 unterscheidet sich von Fig. 1 dadurch, dass ein Verteiler 45 vorgesehen ist, der die Verbindung der einzelnen Geräte zu der Datenleitung 21 übernimmt. An den Verteiler 45 ist das Behandlungsgerät 12 angeschlossen und ein Monitorgerät 46, welches physiologische Parameter des Patienten überwacht, beispielsweise mit einem Sensor 47 für die Blutdruckmessung und einem Sensor 48 für die Pulsmessung. Die Sensordaten werden an den Verteiler 45 übermittelt, der sie an den Arztplatz weiterleitet.

Bei dem Ausführungsbeispiel von Fig. 2 sind die Aufgaben, die am Behandlungsplatz 10 durchgeführt werden, auf verschiedene Geräte aufgeteilt, die sämtlich mit dem Verteiler 45 verbunden sind. Jedes dieser Geräte ist mit einer Vorrichtung zur Personenidentifikation ausgestattet. Auf diese Weise ist die korrekte Zuordnung der Daten dieses Gerätes zu dem jeweiligen Patienten sichergestellt.

## Patentansprüche

1. Medizinisches Behandlungssystem mit
mindestens einem Behandlungsplatz (10) für die Hämodialysebehandlung, der eine Dialysemaschine (12), eine Videokamera (14), einen Bildschirm (24) und eine Gegensprechvorrichtung (16) aufweist, und
mindestens einem Arztplatz (30), der einen Computer (31), einen Bildschirm (32) und eine Gegensprechvorrichtung (34) aufweist,
wobei der Computer (31) des Arztplatzes (30) mit der Dialysemaschine (12) über eine Datenübertragungsverbindung zur Übertragung von Behandlungsdaten kommuniziert,
**dadurch gekennzeichnet, dass**
die Kommunikation der Videokamera (14) des Behandlungsplatzes (10) mit dem Bildschirm (32) des Arztplatzes (30) und die Kommunikation der Gegensprechvorrichtungen (18, 34) von Behandlungsplatz (10) und Arztplatz (30) über die Datenübertragungsverbindung (21, 40, 36), oder über einen dieser fest zugeordneten Kanal, erfolgt,
wobei einzustellende Behandlungsparameter, die von einem Arzt bestimmt werden, einem Pflegepersonal über die Datenübertragungsverbindung mitgeteilt werden, indem sie auf dem Bildschirm (24) des Behandlungsplatzes (10) angezeigt werden.

2. Medizinisches Behandlungssystem nach Anspruch 1, **dadurch gekennzeichnet, dass** an dem Arztplatz (30) eine weitere Videokamera (33) angeordnet ist, die mit dem Bildschirm (24) des Behandlungsplatzes (10) über die Datenübertragungsverbindung (21, 40, 36) oder einen dieser fest zugeordneten Kanal kommuniziert.

3. Medizinisches Behandlungssystem nach Anspruch 1 oder 2, **dadurch gekennzeichnet, dass** an dem Behandlungsplatz (10a) ein Verteiler (45) vorgesehen ist, an den die Dialysemaschine (12), die Videokamera (14), der Bildschirm (24) und die Gegensprechvorrichtung angeschlossen sind und der mit der Datenübertragungsverbindung (21, 40, 36) verbindbar ist.

4. Medizinisches Behandlungssystem nach einem der Ansprüche 1 bis 3, **dadurch gekennzeichnet, dass** die Videosignale der Videokamera (14) und die Audiosignale der Gegensprechvorrichtung (16) in digitalisierter Form über die Übertragungsverbindung (21, 40, 36) übertragen werden.

5. Medizinisches Behandlungssystem nach einem der Ansprüche 1 bis 4, **dadurch gekennzeichnet, dass** die Videokamera (14) des Behandlungsplatzes (10) an der Dialysemaschine (12) angebracht ist.

6. Medizinisches Behandlungssystem nach einem der Ansprüche 1 bis 5, **dadurch gekennzeichnet, dass** die Videokamera (14) des Behandlungsplatzes (10) von dem Arztplatz (30) aus fernsteuerbar ist, um auf verschiedene Ziele gerichtet werden zu können.

7. Medizinisches Behandlungssystem nach Anspruch 6, **dadurch gekennzeichnet, dass** die Videokamera (14) des Behandlungsplatzes (10) eine Vorzugsrichtung aufweist, die sie einnimmt, wenn keine manuelle Steuerung der Richtung der Videokamera vom Arztplatz (30) aus erfolgt.

8. Medizinisches Behandlungssystem nach einem der Ansprüche 1 bis 7, **dadurch gekennzeichnet, dass** am Behandlungsplatz (10) eine Vorrichtung (19) zur Personenidentifikation fest installiert ist, die eine den Patienten kennzeichnende Identifizierung (PID) an einen Computer der Dialysemaschine (12) liefert.

9. Medizinisches Behandlungssystem nach Anspruch 8, **dadurch gekennzeichnet, dass** die Vorrichtung (19) zur Personenidentifikation eine für den Behandlungsplatz (10) verantwortliche Bedienungsperson kennzeichnende Identifizierung an den Computer der Dialysemaschine (12) liefert.

## Claims

1. Medical blood treatment system comprising
at least one treatment station (10) for hemodialysis treatment, which has a dialysis machine (12), a video camera (14), a screen (24) and an intercom device (16), and
at least one doctor's station (30) comprising a computer (31), a screen (32) and an intercom device (34),
wherein the computer (31) of the doctor's station (30) communicates with the dialysis machine (12) via a data transmission link for transmitting treatment data,
**characterized in that**
the communication of the video camera (14) of the treatment station (10) with the screen (32) of the doctor's station (30) and the communication of the intercom devices (18, 34) of the treatment station (10) and the doctor's station (30) takes place via the data transmission link (21, 40, 36), or via a channel permanently assigned thereto,
wherein treatment parameters to be set, which are determined by a doctor, are communicated to nursing staff via the data transmission link by being displayed on the screen (24) of the treatment station (10).

2. Medical blood treatment system according to claim 1, **characterized in that** a further video camera (33) is arranged at the doctor's station (30), which communicates with the screen (24) of the treatment station (10) via the data transmission link (21, 40, 36) or a channel permanently assigned thereto.

3. Medical blood treatment system according to claim 1 or 2, **characterized in that** a distributor (45) is provided at the treatment station (10a), to which the dialysis machine (12), the video camera (14), the screen (24) and the intercom device are connected and which can be connected to the data transmission link (21, 40, 36).

4. Medical blood treatment system according to one of claims 1 to 3, **characterized in that** the video signals of the video camera (14) and the audio signals of the intercom device (16) are transmitted in digitized form via the transmission link (21, 40, 36).

5. Medical blood treatment system according to one of claims 1 to 4, **characterized in that** the video camera (14) of the treatment station (10) is attached to the dialysis machine (12).

6. Medical blood treatment system according to one of claims 1 to 5, **characterized in that** the video camera (14) of the treatment station (10) can be remotely controlled from the doctor's station (30) in order to be directed towards different targets.

7. Medical blood treatment system according to claim 6, **characterized in that** the video camera (14) of the treatment station (10) has a preferred direction which it takes if the direction of the video camera is not manually controlled from the doctor's station (30).

8. Medical blood treatment system according to one of claims 1 to 7, **characterized in that** a device (19) for identifying persons is permanently installed at the treatment station (10), which supplies an identification (PID) characterizing the patient to a computer of the dialysis machine (12).

9. Medical blood treatment system according to claim 8, **characterized in that** the device (19) for identifying persons supplies an identification characterizing the operator responsible for the treatment station (10) to the computer of the dialysis machine (12).

## Revendications

1. Système de traitement médical avec
au moins un emplacement de traitement (10) pour le traitement par hémodialyse, qui présente une machine de dialyse (12), une caméra vidéo (14), un écran (24) et un dispositif de communication bidirectionnelle (16), et
au moins un emplacement pour médecin (30), qui présente un ordinateur (31), un écran (32) et un dispositif de communication bidirectionnelle (34),
dans lequel l'ordinateur (31) de l'emplacement pour médecin (30) communique avec la machine de dialyse (12) par l'intermédiaire d'une liaison de transmission de données pour transmettre des données de traitement,
**caractérisé en ce que**
la communication de la caméra vidéo (14) de l'emplacement de traitement (10) avec l'écran (32) de l'emplacement pour médecin (30) et la communication des dispositifs de communication bidirectionnelle (18, 34) de l'emplacement de traitement (10) et de l'emplacement pour médecin (30) ont lieu par l'intermédiaire de la liaison de transmission de données (21, 40, 36) ou par l'intermédiaire d'un canal associé de manière solidaire à cette dernière,
dans lequel des paramètres de traitement à régler, qui sont définis par un médecin, sont notifiés à un personnel de soin par l'intermédiaire de la liaison de transmission de données **en ce qu'**ils sont affichés sur l'écran (24) de l'emplacement de traitement (10).

2. Système de traitement médical selon la revendication 1, **caractérisé en ce qu'**est disposée au niveau de l'emplacement pour médecin (30) une autre caméra vidéo (33), qui communique avec l'écran (24) de l'emplacement de traitement (10) par l'intermédiaire de la liaison de transmission de données (21, 40, 36) ou d'un canal associé de manière solidaire à cette dernière.

3. Système de traitement médical selon la revendication 1 ou 2, **caractérisé en ce qu'**est prévu au niveau de l'emplacement de traitement (10a) un répartiteur (45), auquel sont raccordés la machine de dialyse (12), la caméra vidéo (14), l'écran (24) et le dispositif de communication bidirectionnelle et qui peut être relié à la liaison de transmission de données (21, 40, 36).

4. Système de traitement médical selon l'une quelconque des revendications 1 à 3, **caractérisé en ce que** les signaux vidéo de la caméra vidéo (14) et les signaux audio du dispositif de communication bidirectionnelle (16) sont transmis sous une forme numérique par l'intermédiaire de la liaison de transmission (21, 40, 36).

5. Système de traitement médical selon l'une quelconque des revendications 1 à 4, **caractérisé en ce que** la caméra vidéo (14) de l'emplacement de traitement (10) est installée au niveau de la machine de dialyse (12).

6. Système de traitement médical selon l'une quelconque des revendications 1 à 5, **caractérisé en ce que** la caméra vidéo (14) de l'emplacement de traitement (10) peut être commandée à distance depuis l'emplacement pour médecin (30), pour pouvoir être dirigée sur différentes cibles.

7. Système de traitement médical selon la revendication 6, **caractérisé en ce que** la caméra vidéo (14) de l'emplacement de traitement (10) présente une direction de prédilection, qu'elle adopte quand aucune commande manuelle de la direction de la caméra vidéo n'a lieu depuis l'emplacement pour médecin (30).

8. Système de traitement médical selon l'une quelconque des revendications 1 à 7, **caractérisé en ce qu'**est installé au niveau de l'emplacement de traitement (10) un dispositif (19) servant à l'identification de personnes, qui fournit une identification caractérisant le patient (PID) à un ordinateur de la machine de dialyse (12).

9. Système de traitement médical selon la revendication 8, **caractérisé en ce que** le dispositif (19) servant à l'identification de personnes fournit à l'ordinateur de la machine de dialyse (12) une identification caractérisant un opérateur en charge de l'emplacement de traitement (10).
